# EUROPEAN PATENT SPECIFICATION

(11) **EP 0 852 506 B1**
(45) Date of publication and mention of the grant of the patent: **14.02.2001**
(21) Application number: 96901092.5
(22) Date of filing: 10.01.1996
(51) Int. Cl.: A61M 5/32

(54) **Disposable medical syringe with retractable needle**
Medizinische Einmalspritze mit zurückziehbarer Nadel
Seringue médicale jetable avec aiguille rétractable

(30) Priority: 25.09.1995 IT CA950014; 21.12.1995 IT MI952715
(43) Date of publication of application: 15.07.1998
(73) Proprietor: Muntoni, Guerino, 09047 Selargius (IT)
(72) Inventor: Muntoni, Guerino, 09047 Selargius (IT)
(74) Representative: Filippi, Remo
(86) International application number: IT9600010
(87) International publication number: WO9711731

(56) References cited:
- EP-A- 0 388 137
- EP-A- 0 636 381
- DE-A- 4 340 082
- US-A- 5 232 458
- US-A- 5 328 475

## Description

The invention concerns disposable syringes for medical use. Medical syringes, namely the hollow cylindrical instruments that include a piston, having at one end a protuberance to which a needle is fitted, are well known for the injection of medicaments, for taking blood samples or for any other purposes.

Similarly well known are the serious diseases which can be transmitted by means of syringes where the needles have been used by persons carrying viruses, especially drug addicts, for injecting drugs.

To overcome these problems syringes with a retractile needle have been devised, but these create considerable problems in use.

For example, the prior patent DE A 4 340 082 discloses a medical syringe comprising a piston with an internal mushroom-shaped projection. When the piston is pushed towards the needle, this projection fits into a cavity in an elastic connection with beak, emerging from a hole in the base of the syringe's cylinder, the needle being applied to the beak.

Therefore on pulling the piston back, said elastic connection will pull the needle inside the cylinder and so prevent its being used again.

The mushroom-shaped projection comprises three sections separated by radial incisions. When the projection is inserted into the mouth of the cavity, the sections are pushed together so that the incisions are closed.

Subject of the invention is a medical syringe with cylinder, piston, and a retractable needle, the needle being fixed to a beak-like body emerging from a hole in the base of the cylinder and being fixed to an elastic block tightly fitted inside said cylinder close to its base.

In said block is a chamber the diameter of whose mouth is less than that of a small head fixed frontwards, by means of an axial stem, to the head of the piston.

The external edges of said small head and mouth are bevelled to facilitate entry, on completion of the injection, of said small head inside the above mentioned chamber, while the internal edges are sharp to prevent the piston becoming detached from the block when it is withdrawn after making the injection.

The volume created between the small head and the head of the piston, once the small head is inserted into the mouth of the chamber of said elastic block, is connected through channels to small holes made on the top of the small head.

The block presents lateral means for ensuring a moderately tight and elastic association to the wall of the cylinder to ensure stability when the needle enters the body of the patient, but without impeding detachment of said block from the bottom of the cylinder due to pull by the piston during its backward stroke after use.

The lateral means of moderately elastic association between the block and the cylinder of the syringe consist of annular ridges, made round the external cylindrical walls of the block, and of annual channels, whose shape is complementary to said ridges, made on the internal wall of the cylinder.

The edges of the annular ridges on the block, facing towards the piston, are rounded or bevelled to permit detachment of the block from the bottom of the cylinder when the piston makes its backward stroke after completing the injection.

The means for reciprocal association between the small head on the piston's main head and the mouth to the chamber, present in the elastic block that supports the needle, are of a simple and effective technical design such as can ensure their safe and perfect operation over time.

The presence of small channels and holes made in the small head joined to the piston head permits transfer of the liquid in the cylinder to the needle.

Immediately after use a simple gesture withdraws the needle safely inside the syringe so that, whether it is handled by specialized persons or by anyone else, there can be no risk of injury or infection.

This invention therefore drastically lessens the danger of infection caused in particular by viruses present in a patient's blood, their natural habitat.

Characteristics and purposes of the invention will be made still clearer by the following examples of its execution illustrated by driagrammatically drawn figures.

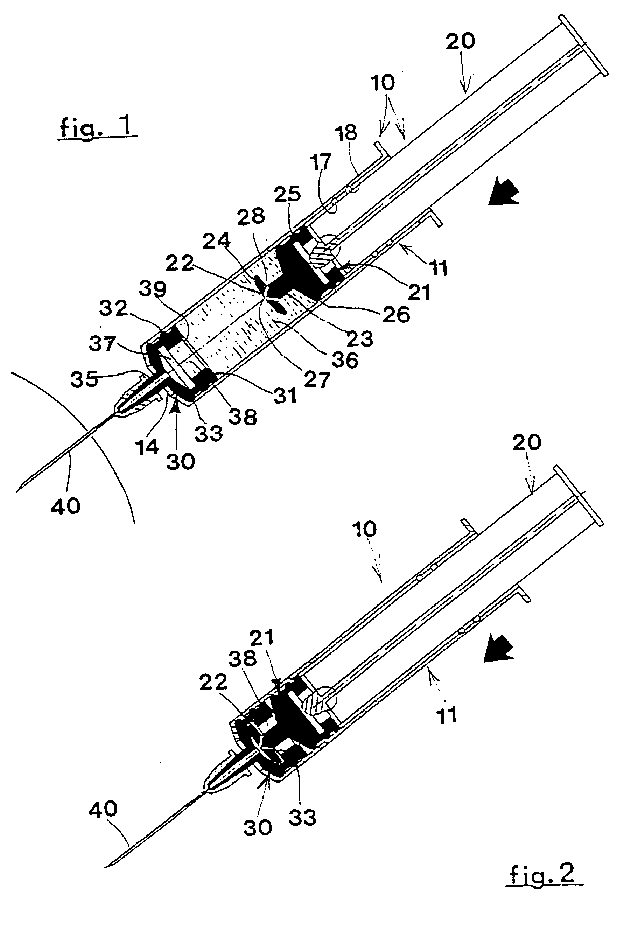

- Fig. 1: Longitudinal section of the syringe ready for use.
- Fig.2: The syringe, longitudinal section, after the injection and when the piston has been pressed right down.
- Fig.3: Longitudinal section of the syringe when the piston is moving back to cause the used needle to enter the syringe.
- Fig.4: Longitudinal section of the syringe when the piston has completed its upward stroke and the needle is entirely inside the syringe.

The syringe 10 comprises the cylinder 11, the piston 20 and the needle-carrying block 30.

On the internal end of the piston is a head 21 with a further small projecting head 22 at the end of a shank 23, said small head having beveled external edges 24.

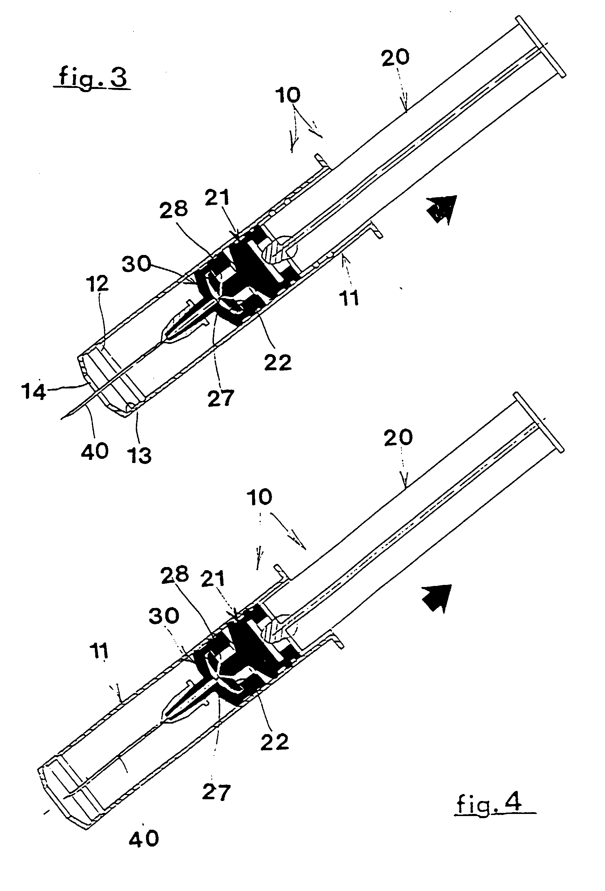

The elastic block 30 exhibits a cylindrical body 31 whose external diameter is substantially equal to the internal diameter of the cylinder of the syringe, and two annular ridges 32,33 that fit into corresponding annular grooves 12, 13 on the wall at the bottom of the cylinder 11 of the syringe (see Fig.3).

On the block 30 is an axial beak-shaped protuberance 35, whose form and size are substantially the same as those of the usual syringes in the same position, for fitting on the needle 40.

Said protuberance 35 emerges from the hole 14 made centrally in the bottom of the cylinder of the syringe.

In the block 30 there is also a central discoid chamber 37 whose diameter is greater than the external diameter of the small head 22 on the piston 20, diameter of the mouth 38 being less than that of the small head.

The external rim of said mouth is beveled 39.

Injection of the liquid 36 having been completed, as with an ordinary syringe, and on giving a further short push to the piston, piston and block will become associated as seen in Fig. 2.

At this point, on pulling the piston back, the block will follow it drawing the needle as well inside the cylinder 11 as seen in Fig.3.

By pulling the piston back still further, the annular ridges 25,26 on the head 21, will fit into the annular grooves 17,18, present close to the external end of said cylinder.

In this way the piston is fixed at the end of its stroke and stabilized in this position with the needle inside the syringe thus preventing its coming out accidentally and therefore its accidentally coming into contact with anyone.

Even in the presence of infected blood maximum protection against infection by contact is given.

The volume between the head 21 of the piston and the piston's small head 22 communicates through holes 27 made on the top of the small head and channels 28.

## Claims

1. Medical syringe (10) with cylinder (11), piston (20), and a retractable needle (40), the needle (40) being fixed to a beak-like body (35), emerging from a hole (14) in the base of the cylinder (11) and being fixed to an elastic block (30) tightly fitted inside said cylinder close to its base, said block (30) presenting a chamber (37) with a mouth (38) of a diameter less than that of a small head (22) fixed frontwards, by an axial stem of a smaller diameter, to the head (21) of the piston (20), said small head and mouth having beveled external edges to facilitate, on completion of the injection, entry of said small head (22) inside the chamber (37), and sharp internal edges to prevent the piston becoming detached from the block, when on completion of the injection, said piston is withdrawn,
characterized in that, the volume created between the small head (22) and the head (21) of the piston (20), once the small head (22) is inserted into the mouth (38) of the chamber (37) of said elastic block (30), is connected through channels (28) to small holes (27) made on the top of said small head (22).

2. Syringe (10) as in claim 1,
characterized in that the block (30) is provided with lateral means for ensuring a moderately elastic tight fitting to the walls of the cylinder (11) to ensure its stability when the needle (40) penetrates the body to be treated but without preventing detachment of said block from the base of the cylinder (11) when the piston (20) is being drawn back after completing the injection.

3. Syringe (10) as in claim 2,
characterized in that the lateral means for moderately elastic association between the block (30) and the cylinder (11) of the syringe consist of annular ridges (32, 33) around the external cylindrical walls of the block (30) and of annular grooves (12, 13) whose form is complementary to said ridges (32, 33) made on the internal walls of the cylinder (11), the edges of the annular ridges (32, 33) around the block facing towards the piston (20) being rounded or beveled to permit detachment of the block from the base of the cylinder (11) when the piston (20) is being drawn back after the injection has been given.

## Patentansprüche

1. Medizinische Injektionsspritze (10) mit Zylinder (11), Kolben und einziehbarer Nadel (40), wobei die Nadel (40) an einen schnabelförmigen Körper (35) befestigt ist, der aus einer Bohrung (14) hervorragt, die am Boden des Zylinders (11) voreingestellt ist, einteilig mit dem elastischen Endmass (30), das, dicht, innenseitig des Zylinders, in der Nähe seines Bodens eingefügt ist und wobei jenes Endmass (30) eine Kammer (37) mit einem Mund (38) aufweist, der einen Durchmesser hat, der kleiner als der eines Köpfchens (22) ist, das vorderseitig über einen Axialschaft befestigt ist, mit einem Durchmesser kleiner als der von Kopf (21) des Kolbens (20) und wobei jenes Köpfchen und jener Mund abgeschrägte Aussenkanten haben um am Abschluss der Injektion den Eintritt des genannten Köpfchens (22) ins Innere der Kammer (37) zu erleichtern und scharfe Innenkanten haben um zu vermeiden, dass der Kolben sich vom Endmass loslöse wenn am Abschluss der Injektion jener Kolben zurückgezogen wird.
Dadurch gekennzeichnet, dass das Volumen, das zwischen dem Köpfchen (22) und dem Kopf (21) des Kolbens (20) geschaffen wird, wenn das Köpfchen (22) in den Mund (38) der Kammer (37) des genannten elastischen Endmasses (30) eingeführt wird, über Kanälchen (28) von kleinen Bohrungen (27), die am Scheitel des genannten Köpfchens (22) ausgeführt worden sind, verbunden wird.

2. Injektionsspritze (10) wie unter Anspruch 1)
dadurch gekennzeichnet, dass das Endmass (30) über Seitenmittel von mässiger elastischer, dichter, Einhakerung an die Wände des Zylinders (11) verfügt um seine Stabilität bei Einführung der Nadel (40) in den Körper, der zu pflegen ist, zu gestatten, ohne jedoch die Trennung des Endmasses worum es hierbei geht, vom Boden selbst des Zylinders (11), - bedingt durch den Zug des Kolbens (20) während des Rücklaufes nach dem Gebrauch - zu verhindern.

3. Injektionsspritze (10) wie unter Anspruch 2),
dadurch gekennzeichnet, dass die Seitenmittel von mässiger elastischer Einhakerung zwischen dem Endmass (30) und dem Zylinder (11) der Injektionsspritze, aus ringförmigen Sicken (32,33) bestehen, um die zylindrischen Aussenwände des Endmasses (30) herum verlegt und aus ringförmigen Kanälen (12,13) bestehen, die in einer Form gestaltet sind, die zu diesen Sicken (32,33) ergänzend ist und die auf der Aussenwand des Zylinders (11) voreingestellt sind, wobei die Kanten der ringförmigen Sicken (32,33) des Endmasses, die zum Kolben (20) hin gerichtet sind, abgerundet oder abgeschägt sind um die Trennung des Endmasses vom Boden des Zylinders (11) beim Rücklauf des Kolbens (20) selbst, nach erfolgter Injektion zu gestatten.

## Revendications

1. Seringue (10) médicale constituée par un cylindre (11), un piston et une aiguille (40) rétractile, l'aiguille (40) étant fixée à un corps (35) muni d'un bec sortant d'un orifice (14) positionné au fond du cylindre (11), solidaire d'un petit bloc (30) élastique étanche, introduit à l'intérieur du cylindre, a proximité de son fond, ledit petit bloc (30) présentant une chambre (37) ayant une ouverture (38) d'un diamètre inférieur par rapport à celui d'une petite tête (22) fixée sur le devant, à l'aide d'une tige axiale ayant un diamètre inférieur, à la tête (21) du piston (20), et ladite petite tête et ouverture ayant des bords externes arrondis, pour faciliter, lorsque la piqûre est terminée, l'entrée de ladite petite tête (22) à l'intérieur de la chambre (37), et des bords internes affilés pour éviter que le piston se décroche du petit bloc lorsque, la piqûre ayant été terminée, ledit piston est retiré.
Caractérisée par le fait que le volume, créé entre la petite tête (22) et la tête (21) du piston (20) lorsque la petite tête (22) est introduite dans l'ouverture (38) de la chambre (37) dudit petit bloc (30) élastique, est relié à travers des petits canaux (28) de petits trous (27) pratiqués sur le sommet de ladite petite tête (22).

2. Seringue (10) comme dans la revendication 1)
Caractérisée par le fait que le petit bloc (30) jouit de moyens latéraux d'accrochage modéré élastique, étanche, aux parois du cylindre (11) pour permettre sa stabilité au moment de l'introduction de l'aiguille (40) dans le corps à soigner, sans toutefois empêcher que le petit bloc susdit se détache du fond du cylindre (11) par effet de la traction du piston (20) pendant la course en arrière après l'emploi.

3. Seringue (10) comme dans la revendication 2)
Caractérisée par le fait que les moyens latéraux d'accrochage-modéré élastique entre le petit bloc (30) et le cylindre (11) de la seringue sont constitués par des reliefs annulaires (32, 33) autour des parois externes cylindriques du petit bloc (30) et par des canaux annulaires (12, 13) d'une forme complémentaire auxdits reliefs (32, 33) positionnés sur la paroi externe du cylindre (11), les bords des reliefs annulaires (32, 33) du petit bloc étant orientés vers le piston (20), arrondis ou épointés pour permettre le détachement du petit bloc du fond du cylindre (11) lorsque, la piqûre étant terminée, ledit piston (20) effectue sa course en arrière.
